(19) Europäisches Patentamt
European Patent Office
Office européen des brevets

(11) **EP 3 922 176 A1**

(12) **EUROPEAN PATENT APPLICATION**
published in accordance with Art. 153(4) EPC

(43) Date of publication:
**15.12.2021 Bulletin 2021/50**

(51) Int Cl.:
*A61B 5/11* (2006.01)   *G06T 7/20* (2017.01)
*G16H 50/20* (2018.01)

(21) Application number: **19914288.6**

(22) Date of filing: **23.08.2019**

(86) International application number:
**PCT/JP2019/032997**

(87) International publication number:
**WO 2020/161947 (13.08.2020 Gazette 2020/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.02.2019 JP 2019018597**

(71) Applicant: **Hitachi, Ltd.**
**Tokyo 100-8280 (JP)**

(72) Inventors:
• **LI Yuan**
  **Tokyo 100-8280 (JP)**

• **PAN Zhang**
  **Beijing 100004 (CN)**
• **MIYAZAKI Kunihiko**
  **Beijing 100004 (CN)**
• **TOYODA Yasutaka**
  **Tokyo 100-8280 (JP)**
• **YUDA Shinya**
  **Tokyo 100-8280 (JP)**

(74) Representative: **MERH-IP Matias Erny Reichl
Hoffmann
Patentanwälte PartG mbB
Paul-Heyse-Strasse 29
80336 München (DE)**

(54) **PHYSICAL HEALTH CONDITION IMAGE ANALYSIS DEVICE, METHOD, AND SYSTEM**

(57) There are provided physical health condition video analysis device, method, and system capable of measuring physical health conditions of a plurality of subjects with high accuracy and low cost by using a video device that can be installed in various environments present in resident areas in daily life. The physical health condition video analysis device includes a 2D information acquisition unit 11 that acquires a two-dimensional video from a camera 10, a 3D information acquisition unit 15 that acquires three-dimensional data from the camera 10 by stereo processing, a target classification unit 12 that classifies targets to be detected from the acquired two-dimensional video, an action specification unit 13 that specifies an action pattern of the target to be detected, a physical feature extraction unit 14 that extracts a physical feature by analyzing the three-dimensional data based on the specified action pattern, and a condition analysis unit 16 that analyzes a physical health condition by using the extracted feature.

*FIG. 1*

**Description**

Technical Field

The present invention relates to physical health condition video analysis device, method, and system that systematically supports, for example, grasping, maintaining, and improving physical health conditions of elderly persons in daily life.

Background Art

In an elderly person care business, various services and facilities are provided to elderly persons who need long-term care. For example, markets for home visitation care, medical services, elderly person homes with long-term care, insurance facilities, medical treatment facilities, group homes, day care, and the like have been very mature. It is obvious that health diagnosis, management, and life support for elderly persons are performed by caregivers. Cooperative support is provided by a large number of experts in order to lead a better life for the elderly persons. For example, in order to maintain health of the elderly persons, support such as a case where a physical therapist visually evaluates a physical condition of the elderly person and gives advice on physical exercise suitable for the condition is performed.
On the other hand, the aging of the population has progressed in recent years, and the market has expanded. According to a survey by the Japan SP Center, the population of the elderly persons (65 years old and over) is estimated to be 36.13 million in 2020. Of these elderly persons, 17.4% are elderly persons who need long-term care, 29.0% are elderly persons who do not need long-term care, and 53.6% are healthy elderly persons. The elderly person care business has expanded a service application range from the care for the elderly persons who need long-term care that have been matured in the related art to services for the elderly persons who do not need long-term care and healthy elderly persons. However, as the needs for long-term care increase, the shortage of resources for experts and long-term care workers has become a social issue. In order to solve the resource shortage, health condition measurement techniques using IoT devices and sensors, and services that support maintenance and improvement of health condition are provided.
The health condition of the elderly person is considered to be three health conditions of a psychological health condition, a physiological health condition, and a physical health condition. Techniques for measuring these health conditions have been widely studied. For example, in NPL 1, as the measurement of the physiological and psychological health conditions, the measurement of a heart rate, a blood pressure, brain waves, and the like using wearable sensors has been reported.
Digital measurement of motions of persons using noncontact sensors has been studied for grasping the physical health condition. For example, a motion capture technique is to digitize motions of a person by attaching markers to the body such as joints of the person, detecting the markers, and processing information on the detected markers. An NEC walking posture measurement system introduced in NPL 2 detects and digitizes an action of walking of a person by extracting positional information and skeleton information of the person by image processing by using Kinect.
In recent years, a deep learning technique has been developed, and it is possible to extract skeletons of a plurality of persons from videos captured by a monocular camera and digitize postures thereof instead of using a method that uses a dedicated active sensor such as Kinect.
A walking motion is processed by using Kinect described in PTL 1 by using these techniques. The skeleton of the person, a position of a landing point, and movement trajectory information are extracted, and thus, it has become possible to provide information for evaluating a walking situation.
It is possible to use these techniques for rehabilitation based on this information. For example, as the rehabilitation, coordinate information of each joint of a walking person can be extracted, and information such as a walking speed and a step width can be displayed without blurring. In addition to the digitization of the motions and postures of the person such as walking, there are techniques related to the digitization of daily actions. For example, information such as motions and positions of a person can be extracted by using a plurality of sensors described in PTL 2, and daily actions of elderly persons for long-term care can be grasped. The information is visualized, and thus, an abnormal action such as falling and slipping is predicted according to the positional information and information on a facility. Accordingly, it is possible to present the predicted abnormal action to a caregiver. For example, when it is determined that the elderly person wants to move to a place where there is a step, it is possible to display assistance for moving the step to the caregiver. It is stated that this risk can be prevented.
PTL 3 describes that high-precision measurement is performed by specifying an action of an elderly person in order to prevent erroneous recognition of a non-elderly person.

Citation List

Patent Literature

> PTL 1: JP 2015-042241 A
> PTL 2: JP 2016-66308 A
> PTL 3: JP 2015-61577 A

Non-Patent Literature

> NPL 1: "Wearable Medical Sensor-based System Design: A Survey", IEEE Transactions on Multi-scale Computing Systems, Vol. 3, No. 2, 2017.
> NPL 2: NEC Walking Posture Measurement System

Summary of Invention

Technical Problem

An object to be solved by the present invention is to measure physical health conditions of a plurality of elderly persons with high accuracy by using a video device that can be installed in various environments present in resident areas.

In recent years, in this field, many inventions have been made to measure a walking style, balance, and the like of elderly person by using Kinect.

These inventions are premised on measurement in a specific environment in order to maintain measurement accuracy. For example, an installation angle of Kinect is horizontal, and an installation height is a height of the elderly person. There are restrictions on the action of the elderly person to be measured. For example, for the measurement of the walking style, the elderly person needs to walk in a straight line with the camera. Thus, in the inventions based on these methods, the measurement in which a measurement environment is specified and the elderly person acts the specified pattern needs to be performed. Thus, when these methods are actually applied to daily life, there is a feeling of resistance of the elderly persons, and inconvenience such as preparation for installation and correspondence of the elderly person is caused.

The inventions based on these methods have a problem that processing cost is high since three-dimensional data is processed in order to maintain the measurement accuracy.

For example, in PTL 3, an action is specified by matching a three-dimensional model of an action desired to be measured in advance with actually acquired three-dimensional data, but in this method, when a plurality of elderly persons and a plurality of actions are measured, since processing cost increases, this technique cannot be realized.

From the above, an object of the present invention is to provide physical health condition video analysis device, method, and system capable of measuring physical health conditions of a plurality of subjects with high accuracy and low cost by using a video device that can be installed in various environments present in resident areas in daily life.

Solution to Problem

From the above, in the present invention, "a physical health condition video analysis device includes a 2D information acquisition unit that acquires a two-dimensional video from a camera, a 3D information acquisition unit that acquires three-dimensional data from the camera by stereo processing, a target classification unit that classifies targets to be detected from the acquired two-dimensional video, an action specification unit that specifies an action pattern of the target to be detected, a physical feature extraction unit that extracts a physical feature by analyzing the three-dimensional data based on the specified action pattern, and a condition analysis unit that analyzes a physical health condition by using the extracted feature".

In the present invention, "a physical health condition video analysis method includes acquiring, as a two-dimensional video, a target to be managed, specifying the target to be managed from the two-dimensional video, and specifying an action pattern of a target to be monitored, acquiring, as a three-dimensional video, the target to be managed, extracting a physical feature by analyzing the three-dimensional video based on the specified action pattern, and; and analyzing a physical health condition by using the extracted feature".

In the present invention, "a physical health condition video analysis system is adopted in which a target to be managed is acquired as a two-dimensional video, the target to be managed is specified from the two-dimensional video and an action pattern of a target to be monitored is specified, the target to be managed is acquired as a three-dimensional video, a physical feature is extracted by analyzing the three-dimensional video based on the specified action pattern, and a physical health condition is analyzed by using the extracted feature, and a location where at least the target to be managed is acquired as the two-dimensional video and a location where the physical health condition is analyzed are connected through communication via a cloud".

Advantageous Effects of Invention

According to the present invention, it is possible to measure physical health conditions of a plurality of subjects with high accuracy and low cost by using a video device that can be installed in various environments existing in a resident area in daily life.

Brief Description of Drawings

[FIG. 1] FIG. 1 is a diagram showing a configuration example of a physical health condition video analysis device according to a first embodiment of the present invention.
[FIG. 2] FIG. 2 is a diagram showing a more detailed configuration example of a target classification unit 12 of FIG. 1.
[FIG. 3] FIG. 3 is a diagram showing action regions (existence regions) of elderly persons in each frame.
[FIG. 4] FIG. 4 is a diagram showing a configuration example of a physical health condition video analysis device according to a second embodiment of the present invention.
[FIG. 5] FIG. 5 is a diagram for describing a processing flow of a physical health condition video analysis method performed by a computer.
[FIG. 6] FIG. 6 is a diagram for describing a processing flow when a physical health condition is evaluated based on a SIDE test of the elderly person by using a stereo camera.
[FIG. 7] FIG. 7 is a diagram showing a configuration example of a physical health condition video analysis device according to a fifth embodiment of the present invention.
[FIG. 8] FIG. 8 is a diagram showing a configuration example of a physical health condition video analysis device according to a sixth embodiment of the present invention.

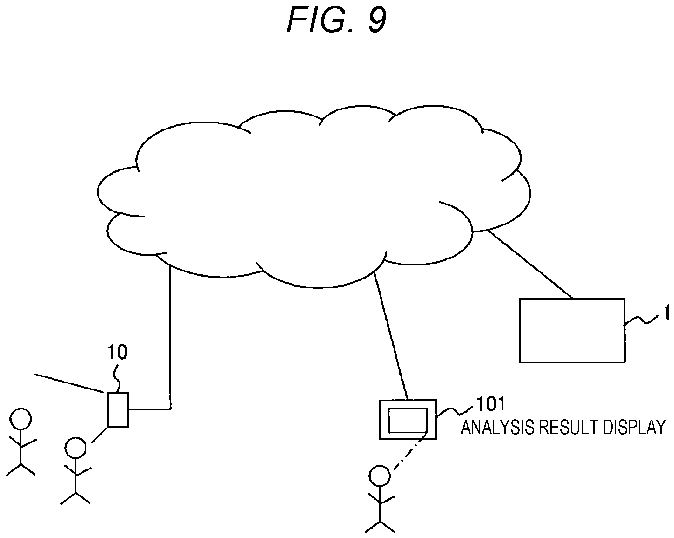

[FIG. 9] FIG. 9 is a diagram showing a configuration example of a physical health condition video analysis device according to a seventh embodiment of the present invention.

Description of Embodiments

Hereinafter, embodiments of the present invention will be described in detail with reference to the drawings. In the following description of the embodiments of physical health condition video analysis device and method according to the present invention, the analysis of the physical health condition of the elderly person will be described as an example.

[First embodiment]

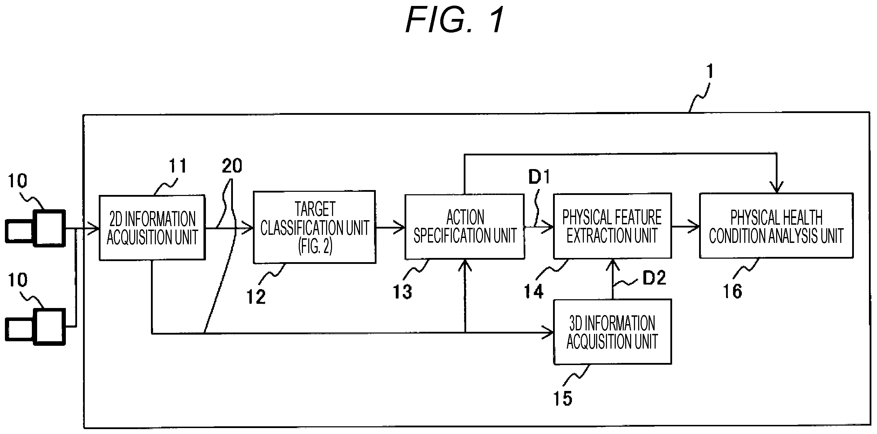

FIG. 1 is a diagram showing a configuration example of a physical health condition video analysis device according to a first embodiment of the present invention. In FIG. 1, 10 is a camera, and 1 is a physical health condition video analysis device according to the first embodiment of the present invention which is a computer device. In the physical health condition video analysis device 1 of FIG. 1, the processing function executed by the calculation unit of the computer device is displayed as a block.
In FIG. 1, first, two-dimensional videos are acquired from the cameras 10. The camera 10 may be a combination of monocular cameras or a stereo camera. A monocular camera includes an image sensor and a lens. The image sensor is a mechanism including an image pick-up device such as complementary metal oxide semiconductor (CMOS) or charge coupled device (CCD). The Lens includes a zoomable lens and a fixed lens. Captured images are stored in a format of BMP, JPGE, or the like. Capturing information includes RGB information.
The stereo camera is a camera that can acquire a depth Depth by simultaneously capturing from multiple viewpoints. Recently, the number of commercially available stereo cameras has been increasing. For example, Intel ReaSense camera has been frequently used. These stereo cameras can simultaneously acquire two-dimensional videos and three-dimensional data. The present invention exemplifies analysis of a physical health condition using the stereo camera constituted by two monocular cameras.
In the physical health condition video analysis device 1 as the computer device, a 2D information acquisition unit 11 first acquires two-dimensional videos from the cameras 10. The acquired videos are stored as images 20 depending on frame rates.

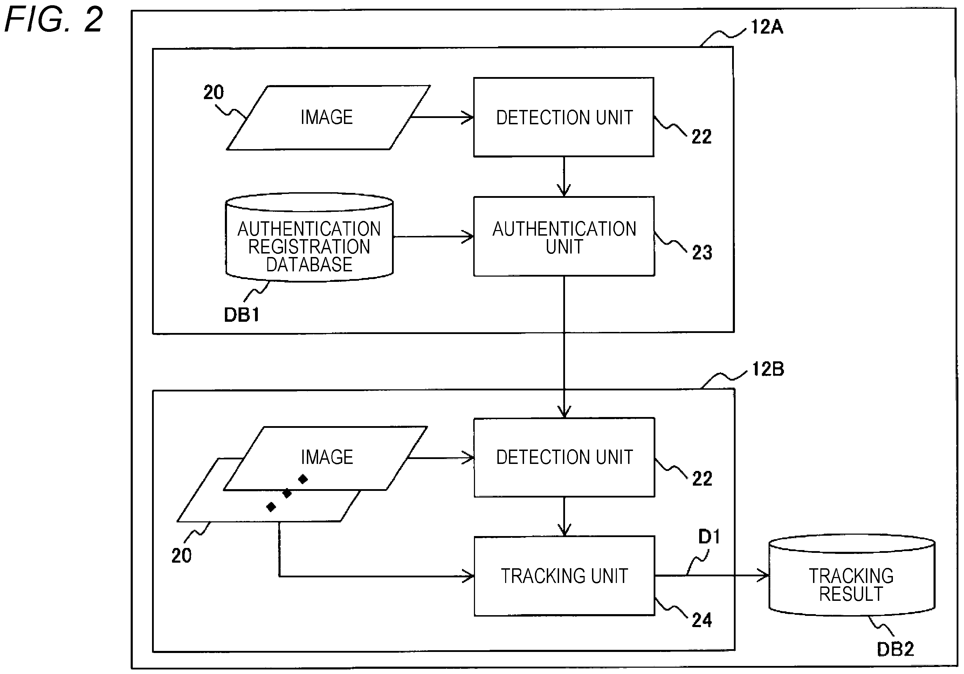

A target classification unit 12 in the physical health condition video analysis device 1 recognizes and classifies, for example, elderly persons as persons from the images 20. In this case, elderly persons to be captured may be one elderly person or a plurality of elderly persons in daily life. Alternatively, the elderly persons may include non-managed persons such as carers. In any case, it is necessary to identify and classify the elderly persons in order to analyze physical health conditions of persons to be managed. The target classification unit 12 in FIG. 2 detects existing persons by using the image 20 acquired from the 2D information acquisition unit 11. That is, persons and subjects other than the persons may be separately grasped from the images, and in this case, the number of persons classified from the image 20 may be one or plural.

FIG. 2 shows a more detailed configuration example of the target classification unit 12. A processing function in the target classification unit 12 is divided into an authentication function 12A and a tracking function 12B. The authentication function 12A is processing of identifying and confirming the elderly persons to be managed, and the tracking function 12B is processing of monitoring the confirmed elderly persons in chronological order.

First, the authentication function 12A of the target classification unit 12 will be described. First, a detection unit 22 detects face and body regions of the elderly persons by using the image 20. As a detection method, various methods such as a matching method of the related art and a recent deep learning technique can be used, and the present invention does not limit this method.

Next, an authentication unit 23 collates with an authentication registration database DB1 registered in advance by using the faces of the detected persons and specifies IDs of the detected elderly persons. When there is not the ID, the elderly person having no ID is registered as an elderly person to be newly managed in the authentication registration database DB1 as necessary. Although this authentication processing may be performed for all frames of the image 20, when a processing rate is taken into consideration, the authentication processing may be performed for a first frame in which the elderly person appears or when the elderly person appears again, and the authentication processing for a continuous subsequent frame may be allotted.

Next, the tracking function 12B of the target classification unit 12 will be described. A detection unit 22 of the tracking function 12B continuously detects and tracks the elderly persons who have been authenticated and managed by the authentication unit 23 of the authentication function 12A in the consecutive frames of the image 20. Although the detection unit 22 of the authentication function 12A and the detection unit 22 of the tracking function 12B are described as separate units in FIG. 2, these detection units may be integrally constructed.

A tracking unit 24 determines whether or not there is the same elderly person in frames detected before and after for the consecutive frames of the image 20. The determination result is stored, as tracking result data D1, in a tracking result database DB2. The tracking result data D1 contains a series of images during this period when there are the elderly persons.

In the present invention, tracking may be ended at a point in time at which the elderly person to be managed is out of a field of view of the fixed camera, or the elderly person to be managed by a camera of which a field of view can be changed may be tracked while changing a camera angle and the like. The point is that a plurality of images can be acquired according to movement of the elderly person and the movement of the elderly person can be captured.

A format of the tracking result data D1 can be determined depending on needs. For simple ones, a frame number and information on the frame in which an elderly person region is specified may be filled out. The authenticated information can be associated with the tracking result data D1.

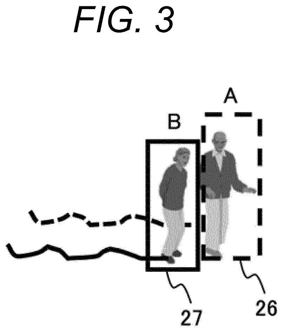

FIG. 3 is a diagram showing action regions (existence regions) of the elderly persons in each frame. According to FIG. 3, the above classification processing is performed, and thus, the IDs of the elderly persons and the action regions (existence regions) in each frame can be grasped for the elderly persons A and B as shown in example results indicated by 26 and 27. These pieces of data in FIG. 3 may be associated and stored, for example, as part of the tracking result data D1 stored in the tracking result database DB2. The tracking result data D1 may be integrated with information on physical health analysis items set by an action specification unit 13 to be described later and stored.

Referring back to FIG. 1, the action specification unit 13 specifies various actions of the elderly persons. Types of actions are different depending on the physical health analysis items. For example, when the physical health analysis item is a walking style and the walking style is analyzed, it is possible to specify a walking action. In the case of rehabilitation, an action desired to be analyzed can be further designated. For example, when knee rehabilitation is performed as the physical health analysis item, it is possible to designate an action specialized for knee bending. Other actions can be added depending on needs. In recent years, there are various deep learning methods for specifying these actions. It is possible to specify various actions of the elderly persons by using these methods. According to recent research, action analysis using CNN and LSTM methods has been known. For example, studies for analyzing actions of persons such as "Deep Local Video Feature for Action Recognition", CVPR, 2017 and "Co-ocuurrence Feature Learning for Skeleton based Action Recognition using Regularized Deep LSTM Networks", AAAI 2016 have been proposed.

The actions specified by the action specification unit 13 can be selected and combined according to the physical health analysis items. For example, in the Time Up and Go (TUG) test, sitting, standing, and walking actions of the elderly persons are specified, and the specified actions are combined depending on the frames. The action specification unit 13 can be positioned as a function of setting the physical health analysis items in the physical health condition video analysis device, and provides management information such as the physical health analysis items for each elderly person to be managed via appropriate input means.

In FIG. 1, a 3D information acquisition unit 15 performs stereo processing by using two left and right two-dimensional images 20 of the cameras 10, and calculates three-dimensional information D2. A calculation method of the three-dimensional information D2 will be described by using the following equation.

[Math. 1]

$$\mathbf{K} = \begin{bmatrix} f & sf & v_c & 0 \\ 0 & af & u_c & 0 \\ 0 & 0 & 1 & 0 \end{bmatrix} \qquad \mathbf{D} = \begin{bmatrix} r_{11} & r_{12} & r_{13} & t_X \\ r_{21} & r_{22} & r_{23} & t_Y \\ r_{31} & r_{32} & r_{33} & t_Z \\ 0 & 0 & 0 & 1 \end{bmatrix} \qquad \cdots (1)$$

Equation (1) is a calculation equation for an internal parameter matrix K and an external parameter matrix D in the camera. Here, f is a focal length, a is an aspect ratio, s is a skew, and (vc, uc) are center coordinates of image coordinates. (r11, r12, r13, r21, r22, r23, r31, r32, r33) indicate orientations of the camera, and (tx, ty, tz) indicate world coordinates of a camera installation position.

When these two parameter matrices K and D and a constant $\lambda$ are used, the image coordinates (u, v) and the world coordinates (X, Y, Z) are associated by a relational expression of Equation (2).

[Math. 2]

$$\lambda \begin{bmatrix} x \\ y \\ 1 \end{bmatrix} = \mathbf{KD} \begin{bmatrix} X \\ Y \\ Z \\ 1 \end{bmatrix} \qquad \cdots (2)$$

In (r11, r12,... r33) indicating the orientations of the camera of external parameters, when Equation (2) is defined by Euler angles, and Equation (2) is represented by three parameters of pan $\theta$, tilt f, and roll $\psi$ which are camera installation angles. Thus, the number of camera parameters required to associate the image coordinates with the world coordinates is 11 which is the total of five internal parameters and six external parameters. Distortion correction and parallelization processing are performed by using these parameters.

Three-dimensional measured values of an object to be measured including a distance, can be calculated as represented in Equation (3).

[Math. 3]

$$\begin{pmatrix} x_l \\ y_l \end{pmatrix} = \frac{f}{Z} \begin{pmatrix} X + \dfrac{B}{2} \\ Y \end{pmatrix} \qquad \begin{pmatrix} x_r \\ y_r \end{pmatrix} = \frac{f}{Z} \begin{pmatrix} X + \dfrac{B}{2} \\ Y \end{pmatrix} \qquad \cdots (3)$$

In Equation (3), (xl, yl) and (xr, yr) are pixel values on the left and right camera images, and after parallelization processing, yl = yr = y. In Equation (3), f is a focal length, B is a baseline, and a distance between the cameras.

Equation (3) is rearranged by using a parallax d. The parallax d on the same three dimension is a difference between images projected on the left and right cameras, and this difference is the parallax on the three dimension. A relationship between the world coordinates and the image coordinates expressed by using the parallax d is as represented in Equation (4).

[Math. 4]

$$\begin{pmatrix} X \\ Y \\ Z \end{pmatrix} = \frac{B}{d} \begin{pmatrix} (x_l + x_r)/2 \\ y \\ f \end{pmatrix} \quad \cdots (4)$$

The 3D information acquisition unit 15 can acquire the three-dimensional information D2 by the above processing flow. The camera such as RealSense can be used as it is when a manufacturer outputs the three-dimensional information D2 as it is.

Referring back to FIG. 1, a physical feature extraction unit 14 will be described. The physical feature extraction unit 14 analyzes features of the specified action for a region of a frame content as shown in FIG. 3 for the action specified by the action specification unit 13 (this information can be obtained from the tracking result data D1 stored in the tracking result database DB2) by using the three-dimensional information D2 calculated by the stereo processing in the 3D information acquisition unit 15. The features defined here are directly related to the physical health condition. For example, smoothness of a standing-up motion is a feature in the case of the standing action, and a position, a speed, a step width, an arm width, and the like are features in the case of the walking action.

A physical health condition analysis unit 16 in FIG. 1 analyzes the physical health condition by using physical features extracted by the physical feature extraction unit 14. It is important to utilize expert knowledge for analysis. Currently, there are various indices and expert evaluation items for evaluating the health conditions of the elderly persons. For example, there is a TUG index with walking ability as an evaluation index. There is a Mini-Mental State Examination (MMSE) evaluation index for evaluating a cognitive function. It is possible to calculate the index based on the features extracted in the previous term.

[Second embodiment]

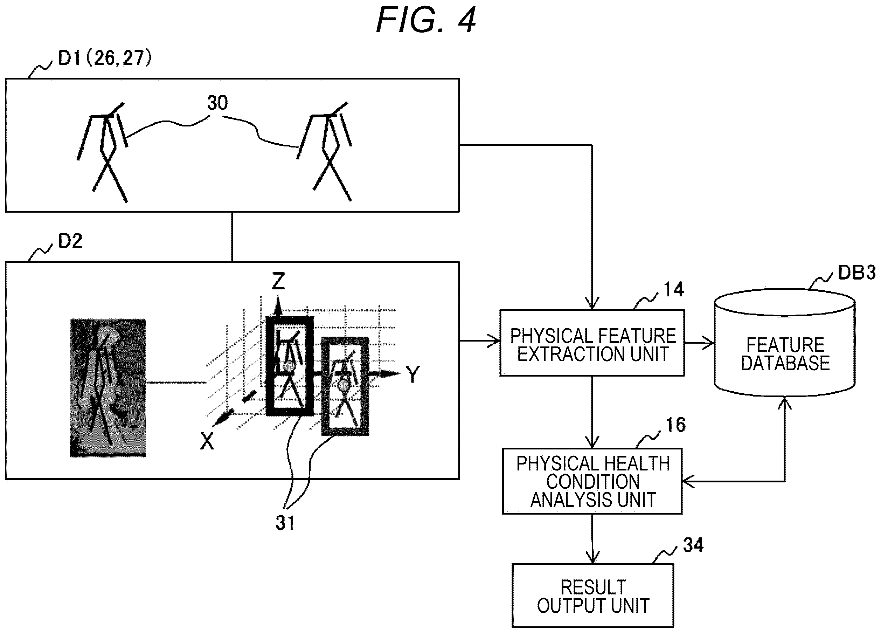

In a second embodiment, an example in which the physical health condition of the elderly person is analyzed by evaluating the walking ability of the elderly person by using the stereo camera which will be described with reference to FIG. 4.

In the second embodiment, the stereo camera includes a camera realized by a combination of sensors capable of outputting a two-dimensional video and three-dimensional information, in addition to a case where the camera is literally the stereo camera. In the present invention, the stereo camera will be described as an example. Since the 2D information acquisition unit 11, the target classification unit 12, the action specification unit 13, and the 3D information acquisition unit 15 are the same as those in the first embodiment, the illustration in FIG. 4 is omitted.

In FIG. 4, the tracking result data D1 from the action specification unit 13 and the like and the three-dimensional information D2 from the 3D information acquisition unit 15 are input to the physical feature extraction unit 14. Of these data and information, the tracking result data D1 includes various kinds of information, but pieces of region information 26 and 27 of the elderly persons illustrated in FIG. 3 are used for the walking action specified from the image in the action specification unit 13. The pieces of region information 26 and 27 include RGB information of the image and pieces of contour information 30 of the persons.

The three-dimensional information D2 from the 3D acquisition information unit 15 is three-dimensional information D2 obtained by performing the stereo processing on the pieces of information 26 and 27 of the elderly persons illustrated in FIG. 3 for the specified region for the walking action specified from the image. According to the three-dimensional information D2, locations of the elderly persons and pieces of practical contour information 31 can be grasped for a three-dimensional space expressed by XYZ axes.

The physical feature extraction unit 14 extracts the features by using the pieces of contour information 31 of the elderly person in the space. For example, a step width can be calculated by measuring a distance in the front and rear frames by using contour position information of the feet. It is possible to calculate necessary features such as speed and acceleration, and it is possible to output the features to a feature database 31.

Hereinafter, a specific calculation method will be described. In the second embodiment, for example, a walking speed and a step width are calculated according to the following flow.

First, skeletons of the elderly persons are extracted from the two-dimensional image D1. A Mask R-CNN method may be used to extract the skeleton. The Mask R-CNN is described in the paper of R. Girshick et al., "Detectron". https://github.com/facebookresearch/detectron, 2018.

Accordingly, it is possible to extract 17 joints in the skeleton of the person. The 17 joints are nose, right eye, left eye, right ear, left ear, right shoulder, left shoulder, right hand, left wrist, right wrist, left hip, right hip, left knee, right knee, left ankle, and right ankle. Image information on 17 joints by the two-dimensional image D1 can be expressed by Equation (5) by using the center coordinates (vc, uc) of the image coordinates.

[Math. 5]

$$feature_{2D}^i = \{[u_1, v_1], \ldots, [u_{17}, v_{17}] \qquad \cdots (5)$$

Equation (5) is an equation of features of 17 joints in the image coordinates. This image information is converted into information on the world coordinates for the same joint by Equation (6) and is expressed as 17 pieces of three-dimensional information. The three-dimensional calculation method can be a stereo method or can be input in advance.

[Math. 6]

$$feature_{3D}^i = \{[x_1, y_1, z_1], \ldots, [x_{17}, y_{17}, z_{17}] \qquad \cdots (6)$$

In order to calculate the speed and the step width, a center point (v18, u18) is calculated by Equation (7) by using 17 joints. The corresponding three-dimensional information is (x, y, z).

[Math. 7]

$$feature_{3D}^i = \{[x_1, y_1, z_1], \ldots, [x_{17}, y_{17}, z_{17}]$$

$$v_{18} = \frac{[(max(v_0, \ldots v_{17}) + min(v_0, \ldots v_{17})]}{2} \qquad \cdots (7)$$

For example, a speed Speed can be calculated by Equation (8) by using the three-dimensional information on the 17 joints. Here, t0 is set to a time interval, that is, 1.5 seconds in the embodiment.

[Math. 8]

$$Speed = \sum_{i=1}^{18} \frac{\sqrt{\left(x_i^{t-t_0} - x_i^t\right)^2 + \left(y_i^{t-t_0} - y_i^t\right)^2 + \left(z_i^{t-t_0} - z_i^t\right)^2}}{18 * t_0} \qquad \cdots (8)$$

A distance d between the left and right feet and a step width length can be calculated by Equation (9). Here, a longest distance in a time zone is calculated as the step width. n is set to 1.0 second.

[Math. 9]

$$dis = \sqrt{(x_{16} - x_{17})^2 + (y_{16} - y_{17})^2 + (z_{16} - z_{17})^2}$$

$$length = max\{d_{t-n}, \ldots, d_{t-1}, d_t\} \qquad \cdots (9)$$

All other necessary features such as acceleration are calculated by using the speed and the step width. In the calculation method, features of other walking styles can be calculated while referring to the paper of Rispens S M, van Schooten K S, Pijnappels M, et al., "Identification of fall risk predictors in daily life measurements: gait characteristics' reliability and association with self-reported fall history", Neurorehabilitation and neural repair, 29 (1): 54-61, 2015.

The physical health condition analysis unit 16 can perform various evaluations such as changes in walking and differences from the same age by using feature data calculated by the above processing. A result output unit 34 outputs the result in a form that can be seen by persons concerned.

[Third embodiment]

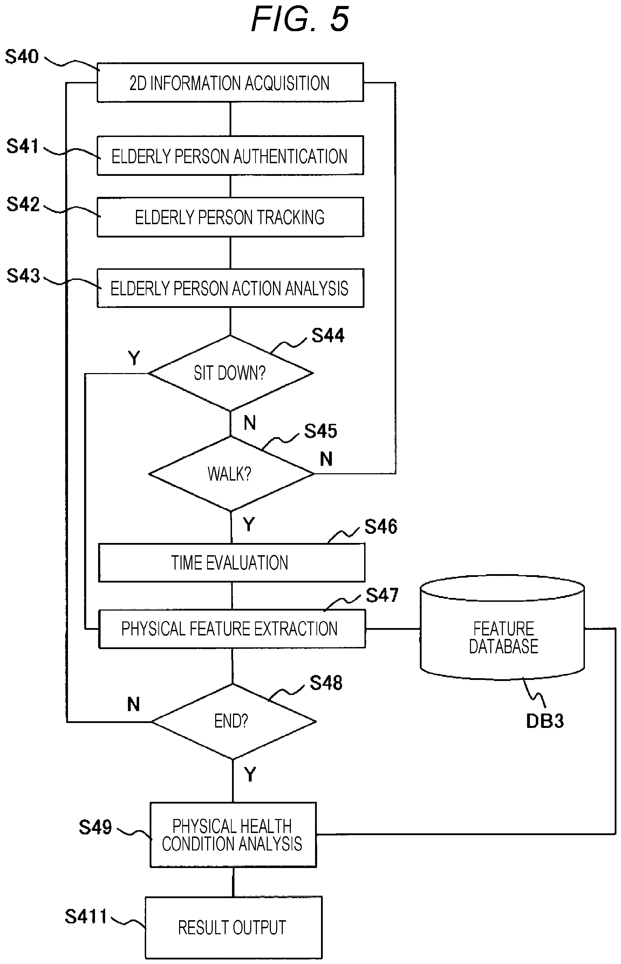

Although the configuration of the physical health condition video analysis device has been described in the first and second embodiments, a physical health condition video analysis method will be described in a third embodiment. In the third embodiment shown in FIG. 5, the physical health condition is evaluated based on the TUG test of the elderly person by using the stereo camera.

Here, the TUG is introduced in Shumway-Cook A, Brauer S, Woollacott M. "Predicting the probability for falls in community-dwelling older adults using the Timed Up & Go Test", Physical Therapy. 2001 Apr; 81(4): 1060-1. Here, the TUG is a test to measure a standing-up time and a time until the elderly person sits down again for a motion of sitting down again after standing up from a chair and walking.

In a flowchart of FIG. 5, first, in processing step S40, the image information 20 is acquired from the 2D information acquisition 40. Elderly person authentication processing step S41, elderly person tracking processing step S42, and elderly person action analysis processing step S43 are performed from the acquired image information. These series of pieces of processing are continuously performed until a series of motions in which the elderly person stands up from the chair, walks, and then sits down again is completed.

When the series of motions up to this point is completed for a specific elderly person, the sitting motion is discriminated in processing step S44, and the walking motion is discriminated in processing step S45. In the case of the sitting action, the physical features when the elderly person sits down are extracted in processing step S47. When the walking action is specified due to the change from the sitting action to the walking action, a time is evaluated in processing step S46. Walking features are extracted.

The features extracted in processing step S47 are as described in the second embodiment. The features extracted in processing step S47 are stored in the feature database DB3. When the acquisition of the image is completed, the physical health condition is analyzed in processing step S49 by using the feature database DB3. Accordingly, it is possible to analyze a time when from the elderly person sits down to when the elderly person sits down again, changes in walking features and sitting features, and differences from a reference value. In processing step S411, the result thereof is output in a form that can be seen by the persons concerned.

Similar to the TUG test, it is possible to combine the sitting and walking actions. It is also possible to change a combination of the actions to be specified based on evaluation indices other than the TUG.

[Fourth embodiment]

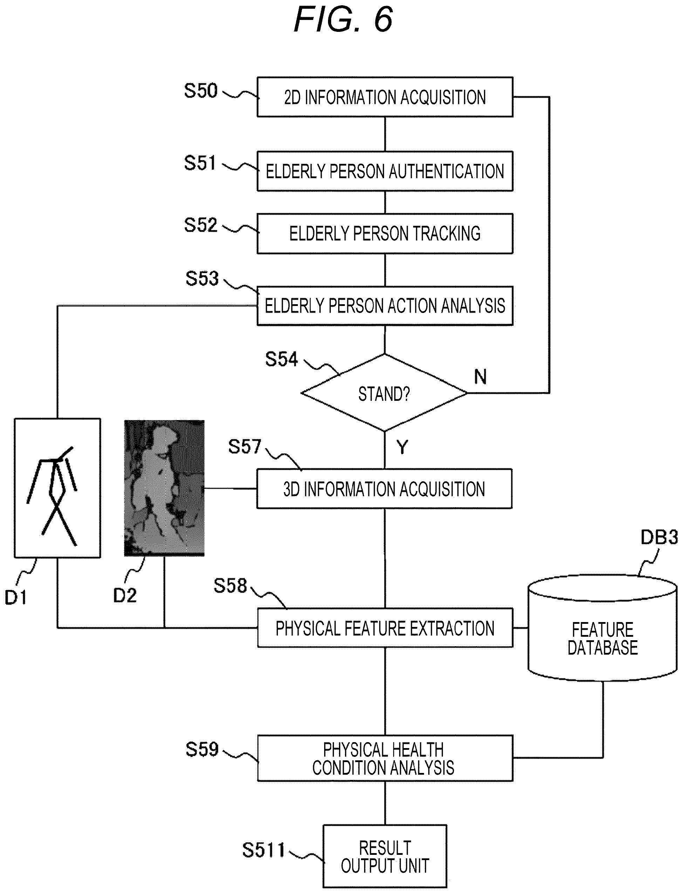

A fourth embodiment will be described with reference to FIG. 6. In the fourth embodiment, the physical health condition of the elderly person is evaluated based on a SIDE test by using the stereo camera.

The SIDE is introduced in Toshio Teranishi, "Standing test for Imbalance and Disequilibrium (SIDE) and its operation", Journal of Japan Fall Prevention Society Vol.4 No.1 2017.

As in the third embodiment, the image information 20 is first acquired from the 2D information acquisition 40 in processing step S50 in the fourth embodiment. Elderly person authentication processing step S51, elderly person tracking processing step S52, and elderly person action analysis processing step S53 are performed from the acquired image information. These series of pieces of processing are continuously performed until a motion in which the elderly person stands up from the chair is completed. In processing step S57, the three-dimensional information D2 during this period is acquired.

In processing step S58, the physical features are extracted by using action information D1 and the three-dimensional information D2 obtained in the processing so far. In processing step S58, the concept of the SIDE was introduced in extracting the physical features, and according to the SIDE, since movement of a lower body is evaluated, it is possible to reduce information on an upper body, and it is possible to improve cost.

For example, a width of the foot can be evaluated by actually calculating a distance between the left and right feet in the three-dimensional space based on the action information D1 (position of the foot) specified on the image. The evaluated features are registered in the feature database DB3. It is possible to analyze the physical health condition of balance of the elderly person based on the evaluation of the SIDE by using the feature database DB3. An analysis result is output in a form that can be seen by the elderly person.

The above-mentioned walking, TUG, and SIDE are evaluation criteria frequently used for caring the elderly persons. Other evaluation criteria can be appropriately performed according to the present invention.

[Fifth embodiment]

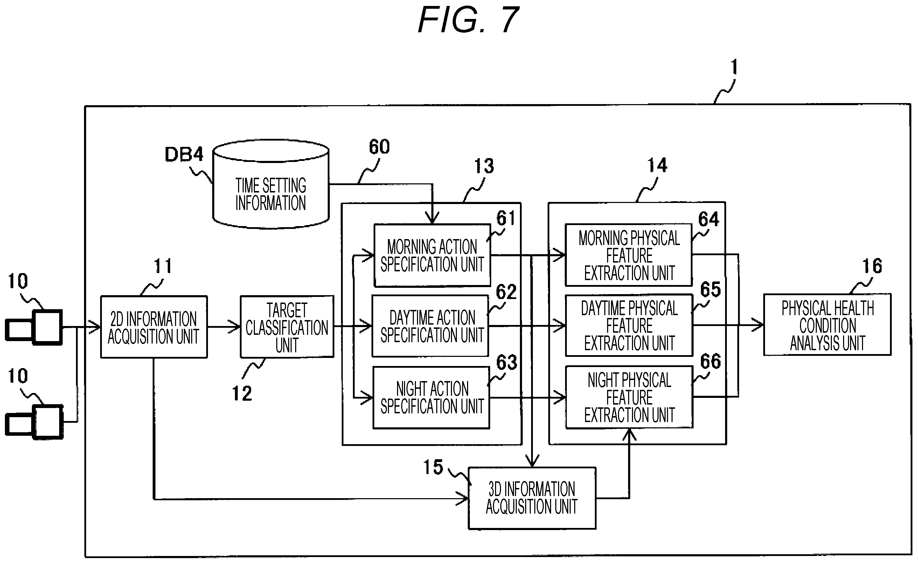

A fifth embodiment will be described with reference to FIG. 7. In the fifth embodiment, the physical health condition is evaluated according to a time. It is possible to adjust the action to be specified and the physical features to be extracted by using time setting information.

In short, it can be said that in a configuration of FIG. 7, the action specification unit 13 is divided into and functions as a morning action specification unit 61, a daytime action specification unit 62, and a night action specification unit 63. Similarly, it can be said that the physical feature extraction unit 14 is divided into and functions as a morning physical feature extraction unit 64, a daytime physical feature extraction unit 65, and a night physical feature extraction unit 66.

In the fifth embodiment, the processing in the action specification unit 13 can be changed between morning, day, and night by using time setting information 60 stored in a time setting information database DB4. For example, the morning action specification unit 61 is performed for the morning time. The physical features are extracted from the morning physical feature extraction unit 64 corresponding to the morning action specification unit. Similarly, in the case of the daytime, the physical features are extracted from the daytime physical feature extraction unit 65 corresponding to the daytime action specification unit 62. In the case of the night, the physical features are extracted from the night physical feature extraction unit 66 corresponding to the night action specification unit 63. Accordingly, the physical health condition analysis unit 68 can be set more flexibly according to the time zone.

The time setting information 60 can be changed by the setting on a user side. Accordingly, more efficient analysis and management of the physical health condition can be realized.

[Sixth embodiment]

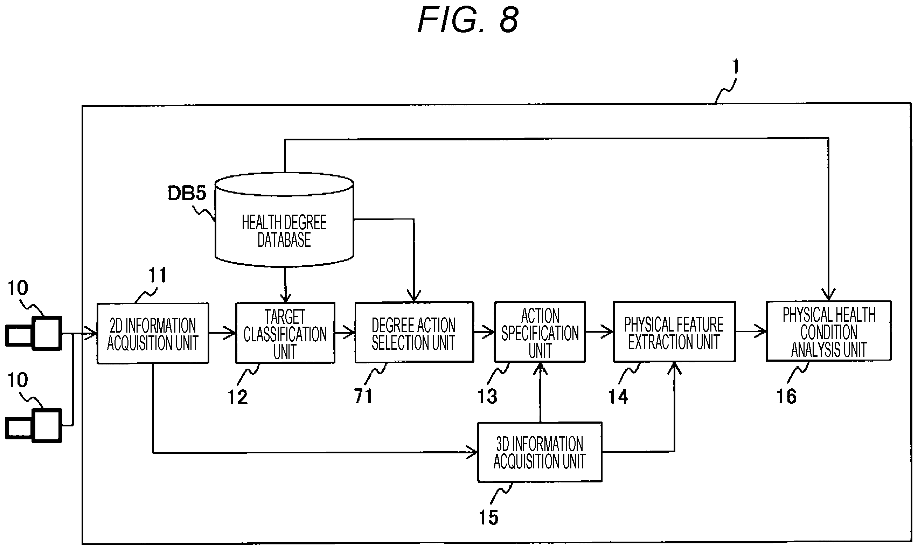

A sixth embodiment will be described with reference to FIG. 8. In the sixth embodiment, the physical health condition is evaluated according to a degree of health of the elderly person. Thus, in FIG. 8, health degree data 70 is preset and stored in a health degree database DB5.

In FIG. 8, the target classification unit 12 can classify the elderly persons. At this time, pieces of information on an age and health of the classified elderly person can be grasped by the health degree data 70 prepared in advance in the health degree database DB5 and authentication information prepared in advance. According to these pieces of information, it is possible to select the action to be specified from a degree action selection unit 71.

A health degree may be set based on expert information. For example, the walking action and the standing action of the SIDE can be selected for an elderly person with a reduced degree of foot in order to evaluate foot rehabilitation. The action specification unit 13 specifies a region related to a foot action depending on the degree of foot. The 3D information acquisition unit 15 calculates the three-dimensional information D2 by performing the stereo processing for the region.

The physical feature extraction unit 14 extracts the physical features by using the output three-dimensional information D2 and the specified foot action information D1. Finally, it is possible to determine whether or not the degree of foot is improved by the foot rehabilitation based on the physical features extracted by the physical health condition analysis unit 14. It is also possible to register a current degree of foot in the health degree database again.

As stated above, it is possible to provide services according to the degree of health of each elderly person.

[Seventh embodiment]

In a seventh embodiment, a desirable embodiment of the physical health condition video analysis device 1 will be described. Here, a physical health condition video analysis system is constructed.

The physical health condition video analysis device 1 illustrated in FIG. 1 is installed in a large hospital as an independent device itself, and can be operated as a device for managing a status of a caring facility in the hospital.

The physical health condition video analysis system is configured to enable remote management as well as the situation according to the present invention. For example, only cameras are installed in depopulated areas where there are few doctors, and elderly persons passing by are observed on a daily basis, and pieces of information on these elderly persons are sent to a cloud. At least a part of physical health condition video analysis devices 1 is installed in the hospital where there are the doctors, physical health analysis by the doctors is performed at an appropriate timing, and results thereof are also sent to the cloud. Here, in the case of the description of at least a part of the physical health condition video analysis devices 1, these pieces of data may be present on any location on the cloud especially around the database. In short, the doctors may analyze the health conditions. Where and how the data is stored, or where and by whom the physical features are performed. In short, it is important for the doctors to be able to store the health condition analysis results on the cloud. It is desirable that the cloud can be accessed by relatives living in remote areas and the like in the elderly persons in the depopulated areas and the health condition analysis results given by the doctors through a handheld tablet, a smartphone 101, and the like can be combined with the action content grasped by the cameras if necessary.

According to the present invention described in detail above, a data processing amount of specified actions of a plurality of subjects by three-dimensional data analysis can be significantly reduced. Accordingly, accurate analysis can be performed by a single image processing chip, and video analysis devices that can be installed in various environments can be provided. Installation costs can be significantly reduced.

Since the stereo processing is performed from the two-dimensional video of the camera and three-dimensional processing is performed, the cameras from which the video and data are acquired are often selected, and restrictions on the installation angle and height are relaxed. Thus, the physical health condition video analysis device can be installed on a daily basis.

From the above, when the services for the elderly persons such as home visitation and caring facilities are provided, it is possible to install the cameras in a place that does not interfere with the lives of the elderly persons. The acquired actions of daily life are analyzed, and thus, it becomes possible to measure the physical health conditions of the elderly persons.

Reference Signs List

| | |
|---|---|
| 10 | camera |
| 11 | 2D information acquisition unit |
| 12 | target classification unit |
| 13 | action specification unit |
| 14 | physical feature extraction unit |
| 15 | 3D information acquisition unit |
| 16 | physical health condition analysis unit |
| 20 | image |
| DB1 | authentication registration database |
| 22 | detection unit |
| 23 | authentication unit |
| 24 | tracking unit |
| DB2 | tracking result database |
| 26 | result of old man A |
| 27 | result of old man B |
| DB3 | feature database |
| DB4 | time setting information database |
| 61 | morning action specification unit |
| 62 | morning physical feature extraction unit |
| 63 | daytime action specification unit |
| 64 | daytime physical feature extraction unit |
| 65 | night action specification unit |
| 66 | night physical feature extraction unit |
| DB5 | health degree database |
| 71 | degree action selection unit |

**Claims**

**1.** A physical health condition video analysis device, comprising:

a 2D information acquisition unit that acquires a two-dimensional video from a camera;
a 3D information acquisition unit that acquires three-dimensional data from the camera by stereo processing;
a target classification unit that classifies targets to be detected from the acquired two-dimensional video;
an action specification unit that specifies an action pattern of the target to be detected;
a physical feature extraction unit that extracts a physical feature by analyzing the three-dimensional data based on the specified action pattern; and
a condition analysis unit that analyzes a physical health condition by using the extracted feature.

2. The physical health condition video analysis device according to claim 1, wherein three-dimensional information is acquired by performing stereo processing on the specified action result.

3. The physical health condition video analysis device according to claim 1, wherein the physical feature is extracted by using the acquired three-dimensional information.

4. The physical health condition video analysis device according to claim 1, wherein physical health conditions of a plurality of elderly persons are analyzed by using the acquired image information and three-dimensional information.

5. The physical health condition video analysis device according to claim 1, wherein an action to be specified in the action specification unit is obtained by combining a plurality of actions.

6. The physical health condition video analysis device according to claim 1, wherein a physical health condition to be analyzed is adjusted according to a time.

7. The physical health condition video analysis device according to claim 1, wherein a physical health condition to be analyzed is adjusted according to a health degree of an elderly person.

8. A physical health condition video analysis method, comprising:

   acquiring, as a two-dimensional video, a target to be managed;
   specifying the target to be managed from the two-dimensional video, and specifying an action pattern of a target to be monitored;
   acquiring, as a three-dimensional video, the target to be managed;
   extracting a physical feature by analyzing the three-dimensional video based on the specified action pattern; and
   analyzing a physical health condition by using the extracted feature.

9. A physical health condition video analysis system in which a target to be managed is acquired as a two-dimensional video, the target to be managed is specified from the two-dimensional video and an action pattern of a target to be monitored is specified, the target to be managed is acquired as a three-dimensional video, a physical feature is extracted by analyzing the three-dimensional video based on the specified action pattern, and a physical health condition is analyzed by using the extracted feature, and
a location where at least the target to be managed is acquired as the two-dimensional video and a location where the physical health condition is analyzed are connected through communication via a cloud.

10. The physical health condition video analysis system according to claim 9, wherein a location where there is a person concerned of the target to be managed is connected through communication via the cloud, and an analysis result of at least the physical health condition is able to be confirmed.

## FIG. 1

# FIG. 2

EP 3 922 176 A1

# FIG. 3

# FIG. 4

# FIG. 5

S40 — 2D INFORMATION ACQUISITION

S41 — ELDERLY PERSON AUTHENTICATION

S42 — ELDERLY PERSON TRACKING

S43 — ELDERLY PERSON ACTION ANALYSIS

S44 — SIT DOWN?  — Y

N

S45 — WALK? — N

Y

S46 — TIME EVALUATION

S47 — PHYSICAL FEATURE EXTRACTION — FEATURE DATABASE (DB3)

S48 — END? — N

Y

S49 — PHYSICAL HEALTH CONDITION ANALYSIS

S411 — RESULT OUTPUT

# FIG. 6

S50 — 2D INFORMATION ACQUISITION

S51 — ELDERLY PERSON AUTHENTICATION

S52 — ELDERLY PERSON TRACKING

S53 — ELDERLY PERSON ACTION ANALYSIS

S54 — STAND? — N

Y

S57 — 3D INFORMATION ACQUISITION

D1

D2

S58 — PHYSICAL FEATURE EXTRACTION

DB3

FEATURE DATABASE

S59 — PHYSICAL HEALTH CONDITION ANALYSIS

S511 — RESULT OUTPUT UNIT

FIG. 7

EP 3 922 176 A1

## FIG. 8

# FIG. 9

101
ANALYSIS RESULT DISPLAY

10

1

### INTERNATIONAL SEARCH REPORT

| International application No. |
|---|
| PCT/JP2019/032997 |

**A. CLASSIFICATION OF SUBJECT MATTER**
Int. Cl. A61B5/11(2006.01)i, G06T7/20(2017.01)i, G16H50/20(2018.01)i

According to International Patent Classification (IPC) or to both national classification and IPC

**B. FIELDS SEARCHED**

Minimum documentation searched (classification system followed by classification symbols)
Int. Cl. A61B5/11, G06T7/20, G16H50/20

Documentation searched other than minimum documentation to the extent that such documents are included in the fields searched
Published examined utility model applications of Japan    1922-1996
Published unexamined utility model applications of Japan  1971-2019
Registered utility model specifications of Japan          1996-2019
Published registered utility model applications of Japan  1994-2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2017-205134 A (NORITSU PRECISION CO., LTD.) 24 November 2017, paragraphs [0036]-[0044], [0070], [0071] & WO 2016/031313 A1 | 1-10 |
| A | JP 2017-521017 A (BLAST MOTION INC.) 27 July 2017, paragraphs [0001], [0015], [0041] & WO 2015/164389 A1, paragraphs [0001], [0014], [0040] & US 2014/0376876 A1 | 1-10 |
| A | US 2013/0289449 A1 (THE CURATORS OF THE UNIVERSITY OF MISSOURI) 31 October 2013, paragraph [0054], fig. 4 & US 2014/0148733 A1 & US 2017/0143240 A1 & US 2019/0029569 A1 | 1-10 |

☐ Further documents are listed in the continuation of Box C.     ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11.09.2019 | 24.09.2019 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2015042241 A
- JP 2016066308 A
- JP 2015061577 A

### Non-patent literature cited in the description

- Wearable Medical Sensor-based System Design: A Survey. *IEEE Transactions on Multi-scale Computing Systems,* 2017, vol. 3 (2
- Deep Local Video Feature for Action Recognition. *CVPR,* 2017
- Co-ocuurrence Feature Learning for Skeleton based Action Recognition using Regularized Deep LSTM Networks. *AAAI,* 2016
- **R. GIRSHICK et al.** *Detectron,* 2018, https://github.com/facebookresearch/detectron
- **RISPENS S M ; VAN SCHOOTEN K S ; PIJNAP-PELS M et al.** Identification of fall risk predictors in daily life measurements: gait characteristics' reliability and association with self-reported fall history. *Neurorehabilitation and neural repair,* 2015, vol. 29 (1), 54-61
- **SHUMWAY-COOK A ; BRAUER S ; WOOLLA-COTT M.** Predicting the probability for falls in community-dwelling older adults using the Timed Up & Go Test. *Physical Therapy,* April 2001, vol. 81 (4), 1060-1
- **TOSHIO TERANISHI.** Standing test for Imbalance and Disequilibrium (SIDE) and its operation. *Journal of Japan Fall Prevention Society,* 2017, vol. 4 (1